# EUROPEAN PATENT APPLICATION

(11) **EP 3 434 611 A2**
(43) Date of publication of application: **30.01.2019**
(21) Application number: 18185946.3
(22) Date of filing: 27.07.2018
(51) Int. Cl.: B65D 6/08, B65D 1/38, A61B 50/00

(54) **PIECE HOLDING SYSTEM FOR INDUSTRIAL USE**

(30) Priority: 28.07.2017 IT 201700087388
(71) Applicant: Ferremi Luca Srl a Socio Unico, 25074 Brescia (IT)
(72) Inventor: FERREMI, LUCA, 25070 BRESCIA (IT)
(74) Representative: Bettello, Pietro

(57) **Abstract**

The invention concerns a piece holding system for industrial use, to be employed in the industrial operating phases related to the transport, storage and cleaning of pieces arranged in an orderly manner, in particular in the industrial sectors of metalworking, mechatronics, precision mechanics, microelectronics, robotics, in the medical and pharmaceutical industries. Said piece holding system provides the use, as a means for containing and/or supporting the pieces, of a container (1) composed of a base and side walls, being provided that on the base, at at least one of the side walls, grids (2, 3) are obtained, where shaped elements are insertable, projecting from said surfaces, having the function of retaining the pieces (10) deposited in said container (1).

## Description

The present invention relates to a piece holding system for industrial use, according to the general part of claim 1.

As is well known, in all industrial sectors, in particular in metal machining, in mechatronics, in precision mechanics, in microelectronics, in robotics, in the medical and pharmaceutical sector are present the processes of assembly, transport, storage, logistics and cleaning of the pieces to be machined.

In the current state of the art, among the various systems used in the industrial operational steps that can be connected, in general, to transporting, storing and cleaning components, a widely used one provides for the use of a planar plate, made of metal or plastic material, wherefrom project some shaped bodies that serve the function of retaining the pieces deposited on the aforesaid plate, so that the pieces are not deposited at random, but rather arranged in an orderly manner and possibly also retained on the aforesaid plate.

In particular the shaped bodies, similar to pegs, known also as "pins", are inserted and retained within cavities obtained on the plate supporting the deposited pieces.

In more detail, the cavities are obtained on the plate so as to form a two-dimensional grid and the pegs are retained on said plate removably, for example with pressure engagements, so that, being able to change and/or replace the type of removable pegs and to position said pegs with a two-dimensional grid space, it is possible to obtain a modular piece holding system, adaptable to the different shapes of the pieces retained on the plate.

By way of example, the patent documents that disclose pegs arranged on a plate, for stacking pieces, are cited:
- EP 3124391 A1 (fig. 1, ref. 2, 13, 14, 15, 16);
- EP 2965998 A1 (fig. 1, ref. 4, 6);
- EP 2570 34 A1 (fig. 1, ref. 2, 3);
- EP 2810887 A1 (fig. 1, ref. 1, 3, 4);
- EP 2860127 A1 (fig. 1, ref. 2, 4);
and the products manufactured by the companies FRIES ("tech-rack variogrid" model) and ALWA ("smartPINS" model).

In practice, the system described above manifests the drawback of having a limited capacity (quantity of pieces contained) on each plate, because the number of pieces is defined by the extension of the planar grid (number of cavities) on which they are deposited and retained, in practice by the number of stacking elements used.

With this constructive solution, in the presence of a high number of pieces (higher than the number that can be contained on the individual grid), it is necessary to use multiple plates which, to facilitate logistics, are positioned mutually superposed, employing appropriate spacers.

In practice, the constructive solution with superposed plates (by way of example, reference is made to the patent documents EP 2952439 A1 (ref. 1, 2, 4), EP 2687454 A1 (ref. 2, 4, 7) and EP 2886482 A1 (ref. 5, 11)) requires considerable bulk, with a non-optimal actual use of the occupied space (low ratio of the number of pieces housed and the volume occupied by the entire piece holding system). Moreover, it is difficult to handle, so that, to facilitate its transport and logistics, it is necessary to use an additional container, normally standard metallic or plastic containers or a containment frame.

The document WO 2014/076394 A1 discloses a piece holding system for industrial use, which uses, as a means for containing and/or supporting the pieces, a container composed of a base and side walls, being provided for that grids are obtained on the base, at at least one of the side walls, where shaped elements are inserted, projecting from said surfaces, having the function of retaining the pieces deposited in said container.

The main purpose of the present invention is to provide a device of this type that is more functional than the device described in said document, and also than the other documents described above.

In particular, a purpose of the present invention is to provide a container of this type that can easily be divided into separate components.

This is obtained by means of a device according to the characterising part of claim 1. The dependent claims relate to particular embodiments of the invention.

Further features of the invention shall be more readily apparent by the description of some of its possible embodiments, provided solely by way of non-limiting example, with the aid of the accompanying drawings, where:
- fig. 1 is a perspective view of the container used in the device of the invention;
- figs. 2, 3 and 4 are orthogonal views of said container;
- fig. 5 shows a possible use of the container of fig. 1;
- figs. 6, 7 show, respectively, exploded and assembled views of a closed container, used in the device of the invention, made with two baskets, referred to in fig. 5;
- figs. 8, 9 and 10 show orthogonal views of the container referred to in fig. 7;
- fig. 11 shows a sectioned view according to the line XI-XI of fig. 9;

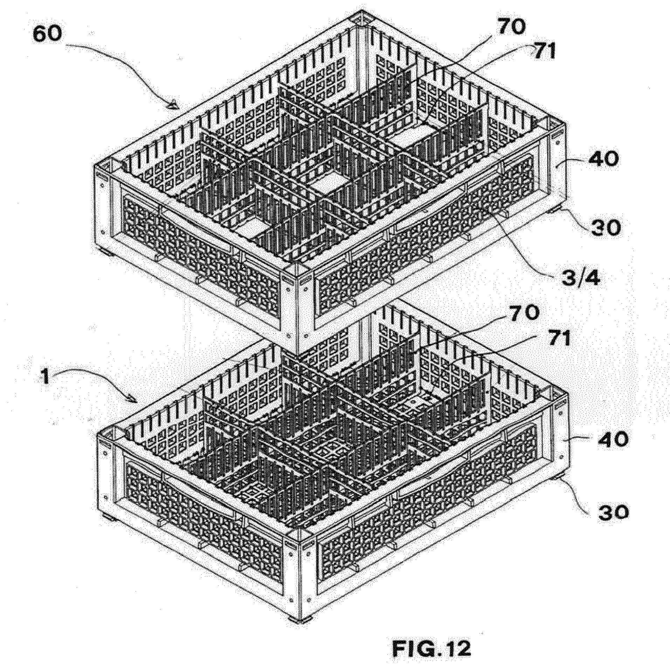

- fig. 12 shows an exploded view of a container, used in the device of the invention, provided with additional frame;

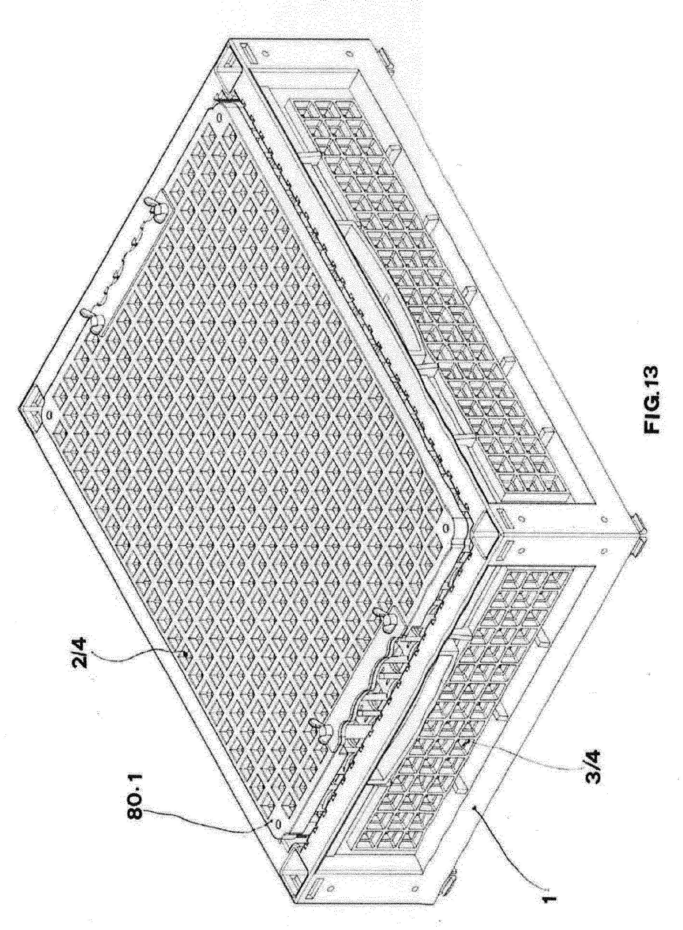

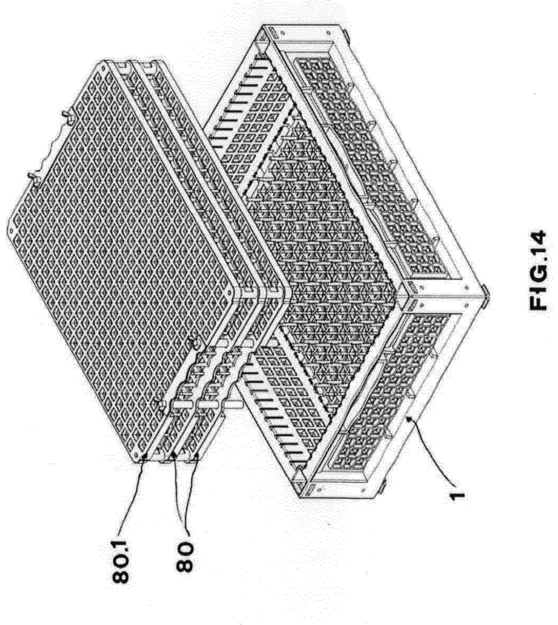

- figs. 13, 14 show, respectively, a working phase view and an exploded view of a container provided with additional intermediate grids according to the invention;
- fig. 15 show a comprehensive view from above of a sealing lid, present in a particular embodiment of the device of the invention, in the open position;
- figs. 16, 17 and 18 show three orthogonal views of said lid, in the open position;
- figs. 19, 20 show two overall axonometric views of the container 1 according to the invention, closed by said lid, as shown in figures 15-18, respectively, with the handle in a lowered and lifted position with respect to the surface of the lid itself.

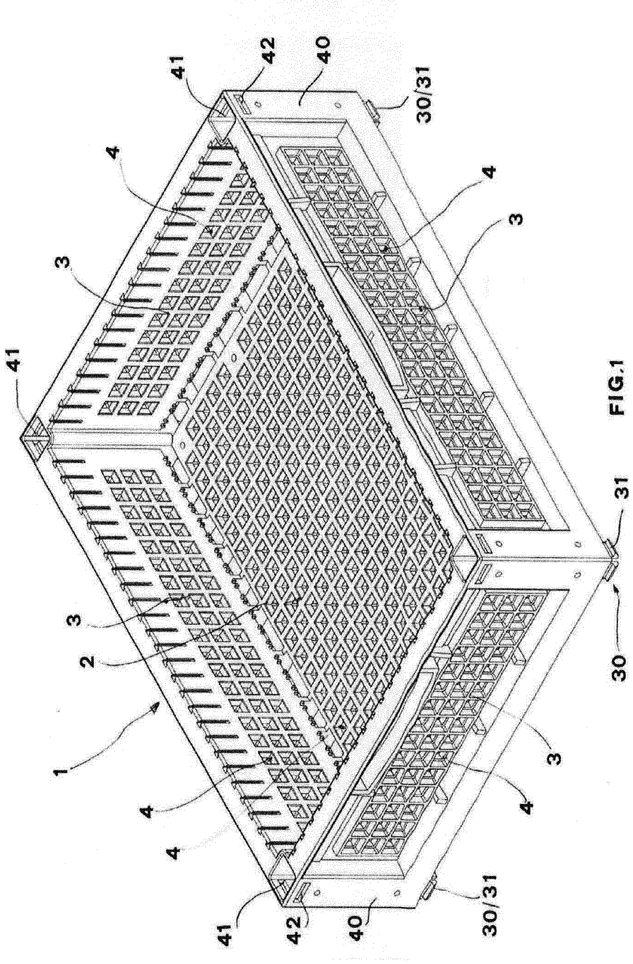

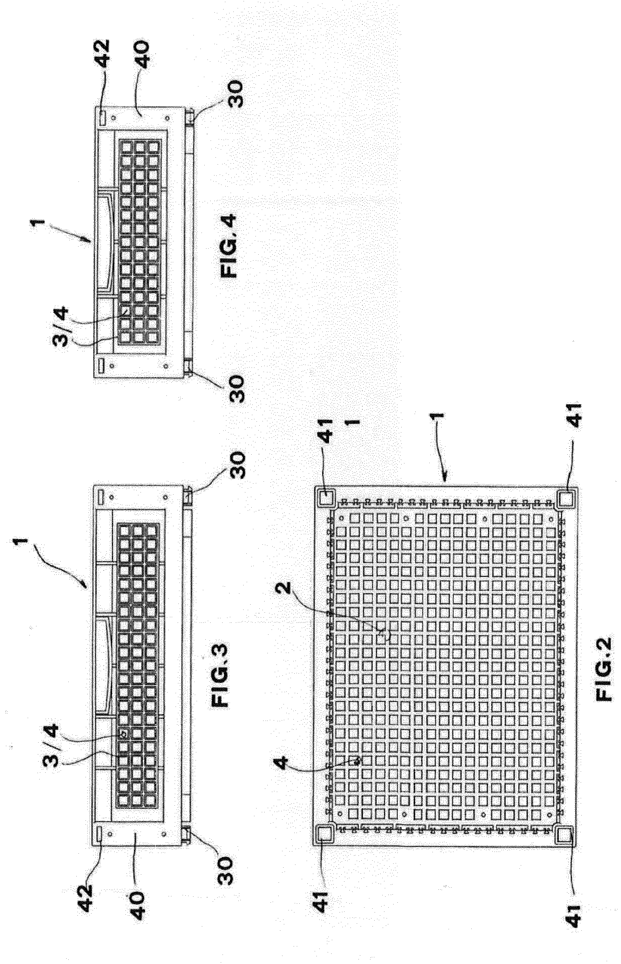

As visible in figs. 1-4, the piece holding system of the invention provides for the use of a container, indicated with the reference numeral 1, shaped as a basket, in which a grid 2 is obtained on the base, while grids 3 are obtained on the walls. In said grids are defined cavities 4, where the ends of pegs 5 and 6, which retain the pieces deposited in said container, are inserted.

Advantageously, the pegs 5, 6 are retained on the grid 2 of the base and on the grids 3 of the walls in a removable manner through pressure inserts, with clips or with other mobile connections, to be able to be differently positioned and/or replaced with others having different conformation, according to the dimensions, to the shape and to the arrangement of the pieces to be retained in the container 1.

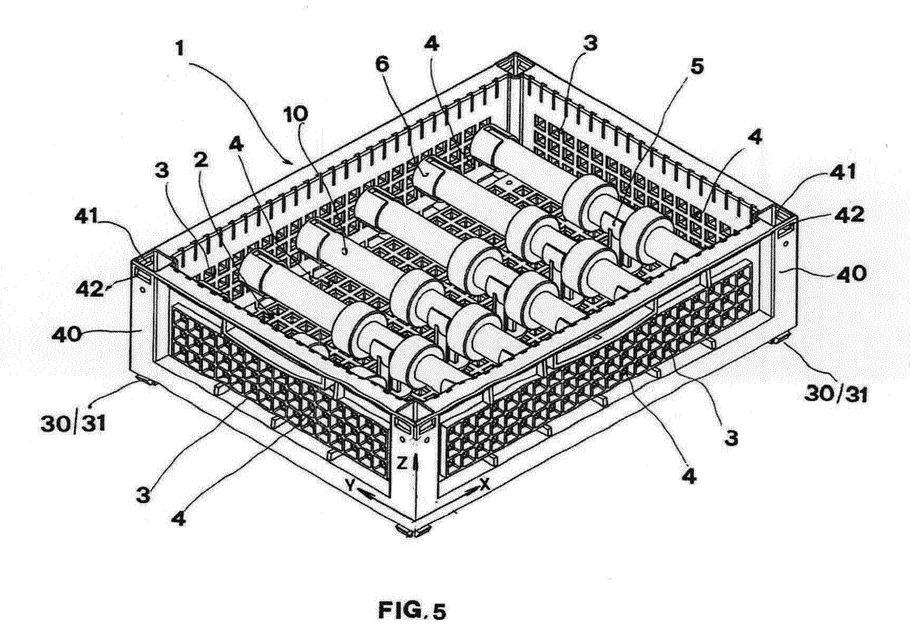

The usefulness of the container of the invention is readily apparent in the use shown in fig. 5, where it is possible to store pieces of considerable length, specifically small shafts 10, which can, advantageously, be arranged with the axis horizontal, i.e. parallel to the base, so they occupy a minimal space in height. Moreover, they are stably blocked in the three mutually orthogonal directions (X, Y, Z), because said pieces are retained, by means of a first series of pegs 5 projecting from the base, in a first plane X-Y and by means of a second series of pegs 6, projecting from the walls, in a second plane X-Z, orthogonal with respect to the first.

Consequently, the possibility of retaining in three-dimensional space the pieces in the container allows to handle the aforesaid container with extreme freedom, even upside down, without any danger that the risk may escape from the container.

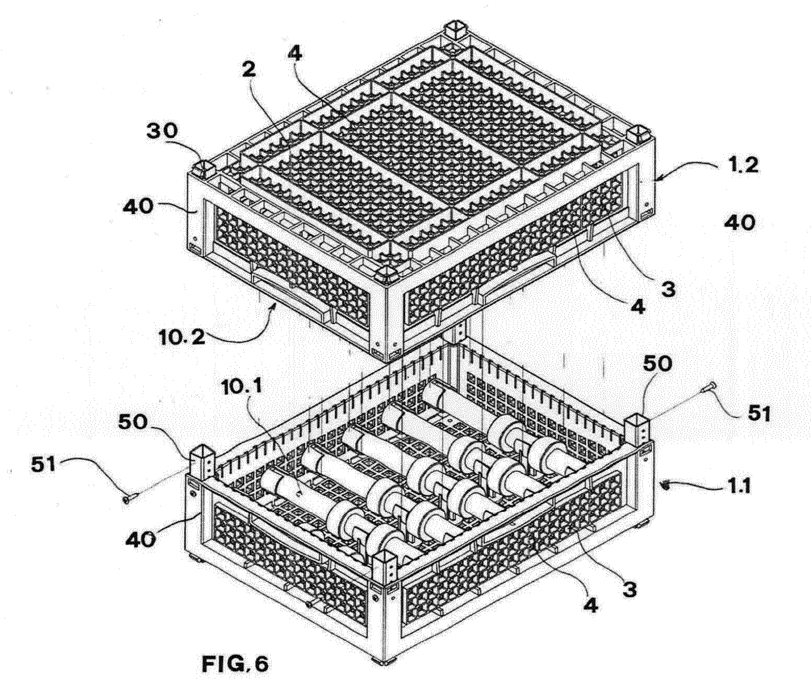

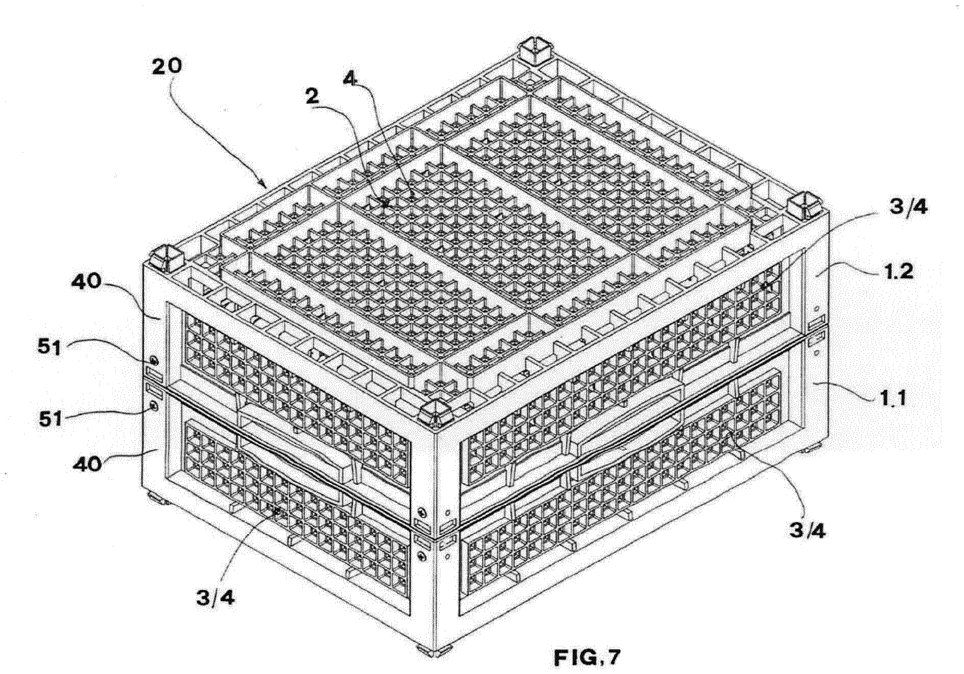

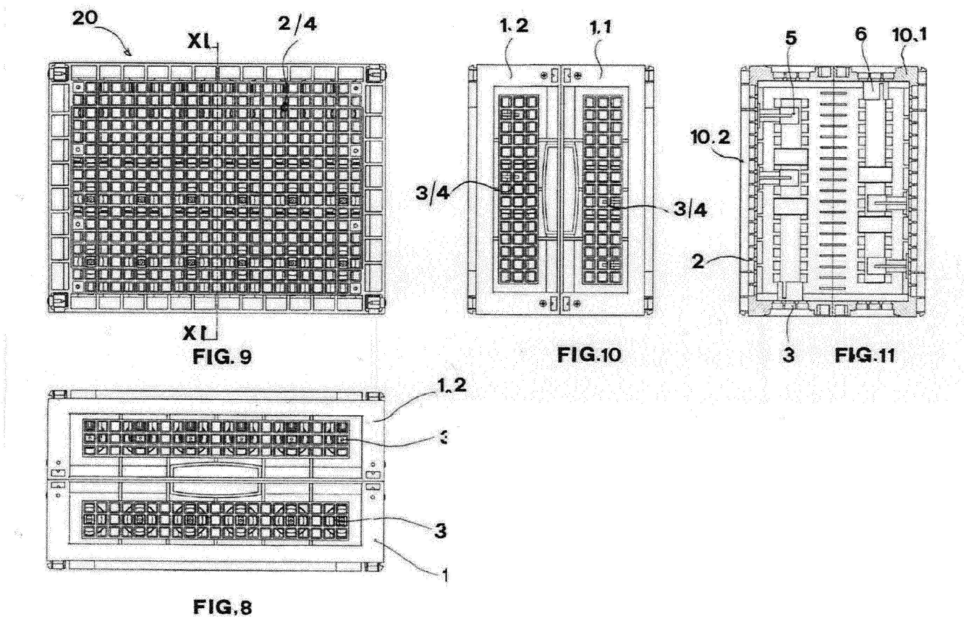

This property allows to use two containers 1.1 and 1.2 stacked mutually opposite, one upside down on the other (see figs. 6, 7) so as to obtain a container closed as a shell 20, so as to have total protection of the objects 10.1 contained in the lower container 1.1 and of the objects 10.2 contained in the upper container 1.2 (see figs. 8-11).

Moreover, to retain the two containers when they are stacked on each other, or to retain on the container 1 an additional frame 60, consisting only of the vertical walls provided with the grids 3 (container without the base), which is used to increase the total volume of the container, there are four connections of the so called "SNAP FIT" type, obtained by providing the container 1 and the additional frame 60 with four feet 30, projecting below the four corner mounts 40 and provided with flexible flaps 31, such that, when the upper container or the additional wall 60 are stacked on the lower container, the aforesaid feet 30 fit into the openings 41, obtained on the top of said corner mounts 40 of the lower container and the flaps 31 of said upper container or of said additional frame 60 fit into the corresponding slits 42, obtained on said corner mount of the lower container.

Moreover, to retain the containers 1.1. and 1.2 when they are stacked in opposite manner, one upside down on the other, the use of pins 50, which are fit on the openings 41 of the corner mounts 40 and, after closing the shell 20, the pins 50 are retained with screws 51.

The container 1 can be provided with one or more additional grids 80 therein, arranged parallel to the base grid 2, so as to create a plurality of shelves in the same space occupied by the aforesaid container and/or to ensure a greater protection of the pieces contained therein, acting as a cover 80.1 and also as dividing walls 70, when it is necessary to ensure, respectively, the closure of compartments 71 for isolation between one piece and the other or the closure of the container.

To facilitate its use, the container 1 will be provided with an alphanumeric numbering of the upper edge and on the base grid (for example a "letter" side - a "number" side) for an easy interpretation of the matrix system and for a simpler management of the inner spaces, and it can be further provided with a label housing, for example with "kanban system", "EAN" codes or other ways, for coding the objects and for an easy interpretation of the content by the end customer.

It should also be considered that, at the end of many mechanical machining operations, the pieces subjected to said operations need to be washed, in order to eliminate the residues and traces of oily substances or dusts. For this reason, the washing and cleansing process becomes essential to separate the various products in the subsequent steps, being them the assembly, the final packaging or the shipping to the customer. The containers containing said pieces, connected in an orderly manner or randomly, are inserted in washing systems manually or automatically. In particular the various industrial washing processes currently used can be carried out statically (the container containing the pieces does not move, remaining in a fixed position), or with tilting (the container is alternatingly inclined by a few degrees), or with a complete continuous rotary motion.

In these two latter cases it is evident that the container must necessarily be closed by a sealing lid, to prevent the products contained therein, during tilting or during the complete rotation, from coming out of the container and falling into the machine causing harmful and dangerous interruptions in the plant.

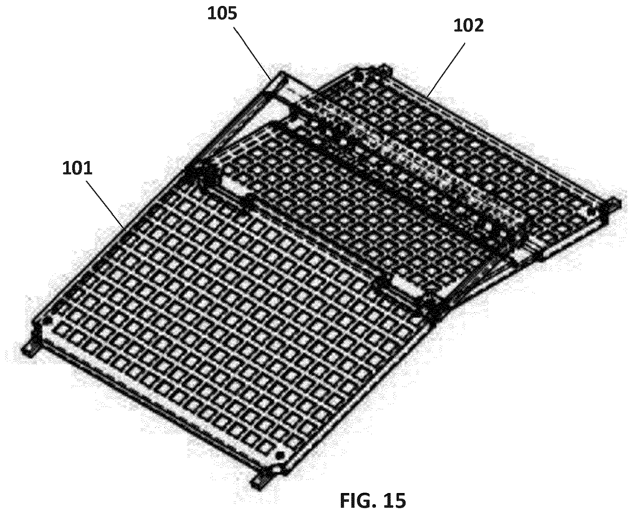

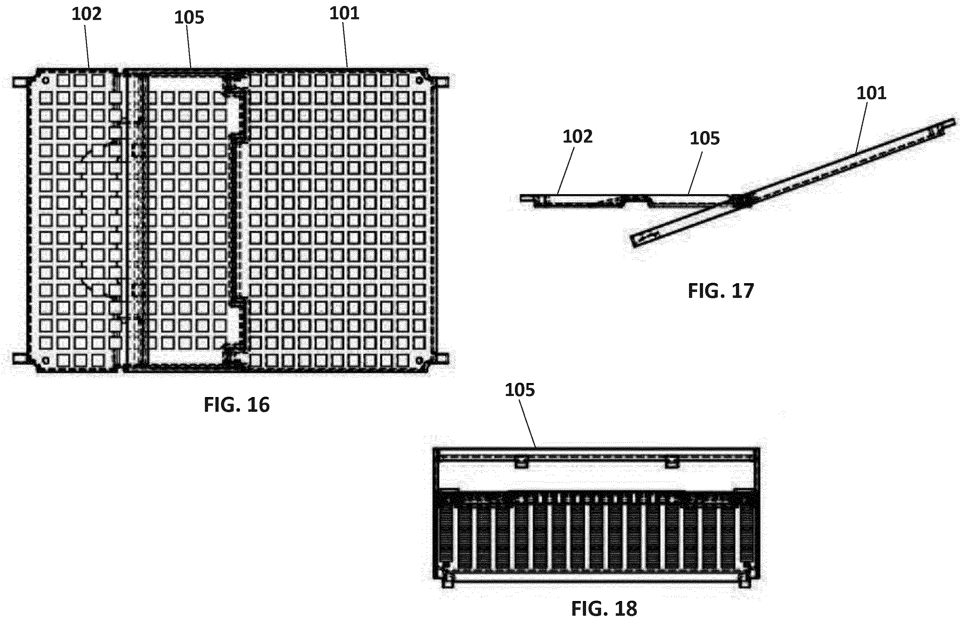

For this purpose, according to a particular embodiment of the device of the invention, a sealing closure lid 100 is provided (see figures 15-20), the term "sealing" meaning that it is adapted to prevent the outwards escape of the elements positioned inside the container.

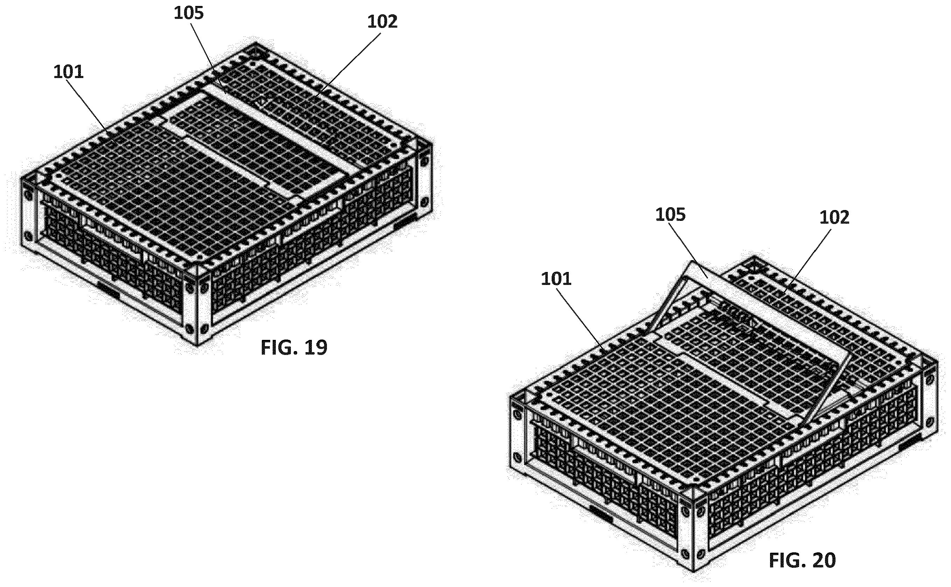

As shown in the figures, said lid consists of two grids 101, 102, mutually hinged by means of pins 100' at their side, which are adapted to be arranged from a mutually coplanar position, as in figures 19 and 20, to a mutually separate position, as shown in figures 15-18. The total dimensions of the two grids are such that they totally occupy the upper opening of the container (not shown in the figures for simplicity) when they are in the coplanar position. Each of the two grids is provided with bayonet projections 103, 104, adapted to be inserted in holes present in the side walls of the container, so as to close it completely.

In addition, a handle 105 is present, hinged to the two grids 101, 102 in correspondence with the pins 100' for the mutual hinging of the two grids.

Moreover, acting on said grids, it is possible to lift the portion thereof whereat they are mutually hinged, thus extracting the bayonet projections 103, 104 of the holes present in the side walls of the container in which they are inserted. In this way the grids move from a position in which they are mutually coplanar, to a position in which they are mutually separate, determining, respectively, the closing and the opening of the container, according to the needs of the user.

It is readily apparent that the possible presence of the sealing closure lid 100 consisting of the two grids 101, 102 is alternative to the presence of the lid 80.1 consisting of an additional grid 80, arranged parallel to the base grid 2.

As shown in the figures, advantageously, a recess 106 is provided in correspondence with one of the two grids 101 or 102, arranged transversely to the grid and extending over its entire transverse dimension, parallel to the side 107 whereat the two grids 101, 102 are mutually hinged. In this way it is evident that it is possible to place the side of the handle 105 that is gripped to move it and hence to act on the two grids 101, 102, so as to position it within the recess 106, so that, as shown in figure 19, in the closed position, the structure comprising the two grids 101, 102 and the handle 105 is positioned on a single plane. In this way, in particular, a plurality of containers provided with this closing system can be stacked without any problem.

Obviously, additional embodiments, dimensions and materials employed of the present invention are possible, provided they are within the inventive concept defined by the claims that follow.

## Claims

1. PIECE HOLDING SYSTEM FOR INDUSTRIAL USE, which provides the use, as a means for containing and/or supporting the pieces, of a container (1) composed of a base and side walls, being provided that on the base, at at least one of the side walls, grids (2, 3) are obtained, where shaped elements are insertable, projecting from said surfaces, having the function of retaining the pieces (10) deposited in said container (1), **said piece holding system being characterised in that** the container (1) is provided at its interior with one or more supplemental grids (80), arranged parallel to the base grid (2), so as to create a plurality of shelves, in order to ensure greater protection of the pieces contained therein and to act as dividing walls (70) and/or as a lid (80.1) when it is necessary to ensure, respectively, the presence of compartments (71) for the isolation between one piece and the other or the closure of the container.

2. PIECE HOLDING SYSTEM, according to claim 1, **characterized in that** it provides for the use of a container (1), shaped as a basket, a grid (2) being obtained in the base thereof and grids (3) being obtained on at least one of the walls thereof, cavities (4) being defined in said grids in which the ends of pegs (5, 6) are to be inserted, such pegs retaining the pieces (10) deposited in said container.

3. PIECE HOLDING SYSTEM, according to one or more of the preceding claims, **characterized in that** it determines a three-dimensional positioning of the pieces (10), which are stably blocked in three mutually orthogonal directions (X, Y, Z), said pieces being retained by means of a first series of pegs (5), projecting from the base, in a first plane (X-Y) and by means of a second series of pegs (6), projecting from the walls, in a second plane (X-Z), orthogonal with respect to the first.

4. PIECE HOLDING SYSTEM, according to one or more of the preceding claims, **characterized in that** it provides for the use of a frame (60) solely composed of vertical walls provided with grids (3) with cavities (4), that is stacked on top of the base container (1) to increase its volume.

5. PIECE HOLDING SYSTEM, according to claim 4, **characterized in that,** in order to retain two containers when they are stacked one above the other, or to retain the frame (60) on the base container (1), four connections of the so called "SNAP FIT" type, obtained by providing the container (1) and the frame (60) with four feet (30), projecting below the four corner mounts (40) and provided with flexible flaps (31), such that, when the upper container is stacked on the lower container, or the frame (60) is stacked on the container below, the aforesaid feet (30) fit into openings (41) obtained on the top of said corner mounts (40) of the lower container and the flaps (31) of said upper container or of said frame fit into corresponding slits (42), obtained on said corner mount of the lower container.

6. PIECE HOLDING SYSTEM, according to one or more of the preceding claims, **characterized in that** it employs two opposing containers (1.1, 1.2), one turned upside down on the other, so as to obtain a final container closed like a shell (20).

7. PIECE HOLDING SYSTEM, according to claim 5, **characterized in that,** in order to retain the opposing stacked containers (1.1, 1.2), the use of pins (50) is provided for, such pins being fit on the openings (41) of the corner mounts (40) and, after closing the aforesaid two containers like a shell (20), said pins (50) are retained by means of screws (51).

8. PIECE HOLDING SYSTEM, according to one or more of the preceding claims, **characterized in that** the pegs (5, 6) are removably retained on the grids (2) of the base and on the grids (3) of the walls, by means of pressure inserts, clip inserts, or with other mobile connections, to be differently positioned and/or replaced with other ones of different conformation, depending on the size, the shape and the arrangement of the pieces to be retained in the container (1).

9. PIECE HOLDING SYSTEM, according to one or more of the preceding claims, **characterized in that** the container (1) is provided with an alphanumeric numbering of the upper edge and on the base grid (for example a "letter" side - a "number" side) for an easy interpretation of the matrix system and for a simpler management of inner spaces, said container being further provided with a label housing, for example with "kanban system", "EAN codes" or other ways, for coding the objects and for an easy interpretation of the content by the end customer.

10. PIECE HOLDING SYSTEM, according to one or more of the preceding claims, **characterized in that** it provides for the presence of a sealing closure lid (100), adapted to prevent the outwards escape of the elements positioned inside the container, consisting of two grids (101, 102) mutually hinged in pins (100') at a side thereof, able to be arranged from a mutually coplanar position, to a mutually separate position, the overall dimensions of the two grids being such that they totally occupy the upper opening of the container (1), when they are in coplanar position, each of said grids being provided with bayonet projections (103, 104) able to be inserted into holes present in the side walls of the container, in order to close it completely, the presence of a handle (105) being provided, hinged to the two grids (101, 102) at the pins (100') for the mutual hinging of the two grids (101, 102), it being provided that acting on said grids (101, 102) it is possible to lift the portions of the grids (101, 102) in correspondence of which they are mutually hinged, extracting the bayonet projections (103, 104) of the holes in which they are inserted, thus passing from a position in which they are mutually coplanar, to a position in which they are mutually separate, determining, respectively, the closure and the opening of the container, depending on the needs of the user.

11. PIECE HOLDING SYSTEM, according to claim 10, **characterized in that** it comprises a recess (106) at one of the two grids (101 or 102), arranged transversely thereto and extending over its entire transverse dimension, parallel to the side (107) in correspondence of which the two grids (101, 102) are mutually hinged in pins (100'), within said recess (106), it being possible to place the side of the handle (105) that is gripped to move it, so as to position it within said recess (106), so that, in the closed position, the structure comprising the two grids (101, 102) and the handle (105) is positioned on a single plane.
